(19) **Europäisches Patentamt European Patent Office Office européen des brevets**

(11) **EP 4 232 811 B1**

(12) **FASCICULE DE BREVET EUROPEEN**

(45) Date de publication et mention de la délivrance du brevet:
**10.09.2025 Bulletin 2025/37**

(21) Numéro de dépôt: **21798663.7**

(22) Date de dépôt: **22.10.2021**

(51) Classification Internationale des Brevets (IPC):
***G01N 29/04*** *(2006.01)* ***G01N 29/34*** *(2006.01)*

(52) Classification Coopérative des Brevets (CPC):
**G01N 29/045; A61B 8/4483; A61B 8/485; G01N 29/348;** G01N 2291/02475; G01N 2291/02818; G01N 2291/106

(86) Numéro de dépôt international:
**PCT/EP2021/079332**

(87) Numéro de publication internationale:
**WO 2022/084502 (28.04.2022 Gazette 2022/17)**

(54) **SONDE DE MESURE DE PROPRIÉTÉS VISCOÉLASTIQUES D'UN MILIEU D'INTÉRÊT**

SONDE ZUR MESSUNG DER VISKOELASTISCHEN EIGENSCHAFTEN EINES BESTIMMTEN MEDIUMS

PROBE FOR MEASURING VISCOELASTIC PROPERTIES OF A MEDIUM OF INTEREST

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.10.2020 FR 2010857**

(43) Date de publication de la demande:
**30.08.2023 Bulletin 2023/35**

(73) Titulaire: **E-Scopics**
**13090 Aix-en-Provence (FR)**

(72) Inventeurs:
• **MAURICE, François**
**83300 DRAGUIGNAN (FR)**
• **BOUSCASSE, Pascal**
**13610 SAINT-ESTEVE-JANSON (FR)**
• **COHEN-BACRIE, Claude**
**13090 AIX-EN-PROVENCE (FR)**
• **WINTZENRIETH, Frederic**
**13100 AIX-EN-PROVENCE (FR)**

• **BESSON, Adrien**
**13007 MARSEILLE (FR)**

(74) Mandataire: **Be IP**
**Cabinet LTL SAS**
**Centre d'Entreprise et d'Innovation**
**56, Bd Niels Bohr**
**CS 52132**
**69603 Villeurbanne Cedex (FR)**

(56) Documents cités:
**EP-A1- 3 315 074 WO-A1-2010/007234**

• **SANDRIN L ET AL: "Shear modulus imaging with 2-D transient elastography", IEEE TRANSACTIONS ON ULTRASONICS, FERROELECTRICS AND FREQUENCY CONTROL, IEEE, US, vol. 49, no. 4, 1 April 2002 (2002-04-01), pages 426 - 435, XP011368258, ISSN: 0885-3010, DOI: 10.1109/58.996560**

**Description**

## DOMAINE DE L'INVENTION

La présente invention concerne le domaine technique général de l'imagerie d'un objet cible, ou d'un milieu diffus tel qu'un tissu biologique humain ou animal.

Plus précisément, la présente invention concerne un dispositif et un procédé pour mesurer des propriétés viscoélastiques d'un tissu biologique d'intérêt.

Elle s'applique en particulier, mais non exclusivement, à la mesure de paramètres de viscoélasticité du foie d'un humain ou d'un animal, cette mesure étant corrélée à la quantité de fibrose présente dans le foie.

## ARRIERE PLAN DE L'INVENTION

Afin de mesurer les propriétés viscoélastiques tissulaires, il est connu d'utiliser l'élastographie par onde de cisaillement.

Cette technique consiste à mesurer la vitesse de propagation d'une onde de cisaillement dans un tissu, cette vitesse étant directement reliée aux propriétés viscoélastiques du tissu analysé.

L'onde de cisaillement peut être engendrée :

- soit par une sollicitation mécanique,
- soit par une sollicitation acoustique.

### 1. Génération mécanique de l'onde de cisaillement

Pour estimer les propriétés viscoélastiques d'un tissu, il a déjà été proposé un appareil médical d'élastographie impulsionnelle ultrasonore, appelé Fibroscan®.

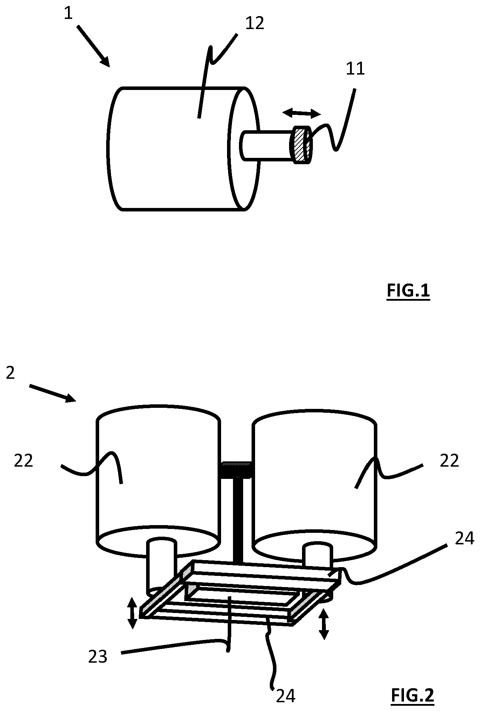

En référence à la figure 1, cet appareil 1 comprend :

- un transducteur ultrasonore 11 pour l'émission d'ondes ultrasonores et l'acquisition d'échos,
- un actionneur électrodynamique 12 formant vibreur,
- un boitier (non représenté) contenant l'actionneur électrodynamique 12.

Le transducteur 11 est fixé à l'extrémité de l'actionneur électrodynamique 12. L'actionneur électrodynamique 12 permet de faire vibrer le transducteur 11 pour générer une onde de cisaillement. Le principe de fonctionnement de l'appareil médical 1 d'élastographie impulsionnelle est le suivant.

L'actionneur électrodynamique 12 est activé pour induire la mise en mouvement du transducteur 11 et générer une onde de cisaillement basse fréquence dans le tissu à analyser. Durant la propagation de l'onde de cisaillement basse fréquence, le transducteur 11 émet et reçoit des ondes ultrasonores haute fréquence afin de permettre l'étude de la propagation de l'onde de cisaillement basse fréquence.

Le mode de génération de l'onde de cisaillement proposé ci-dessus est relativement efficace dans la transmission de l'énergie mécanique au tissu, puisque le transducteur ultrasonore 11, au contact du tissu, est directement mis en mouvement par l'actionneur électrodynamique 12 tenu en main par l'utilisateur.

Toutefois, un inconvénient de cette technologie est qu'elle est inadaptée à la cartographie bidimensionnelle (2D) d'un tissu. En effet, la cartographie 2D d'un tissu nécessite l'utilisation d'un réseau de transducteurs ultrasonores. Or, il est difficile (voir impossible) de fixer un tel réseau de transducteurs à l'extrémité d'un actionneur électrodynamique du fait :

- de ses dimensions d'une part (qui peuvent être de 60x15mm), et
- du faisceau de câbles reliés aux transducteurs du réseau d'autre part.

Il a également été proposé (dans le document intitulé « Shear Modulus Imaging with 2-D Transient Elastography » de Sandrin publié en 2002) de dissocier l'excitation mécanique de la sonde ultrasonore. Notamment, la figure 2 illustre un système 2 d'élastographie impulsionnelle incluant :

- des moyens d'excitation mécanique (formant vibreur) pour la génération d'une onde élastique de cisaillement basse fréquence dans le tissu, et
- des moyens de génération d'ondes ultrasonores haute fréquence pour imager le tissu, les moyens de génération d'ondes étant dissociés des moyens d'excitation mécanique.

En particulier, les moyens d'excitation mécanique du système comportent deux tringles 24 dont les mouvements sont contrôlés par deux électro-vibrateurs magnétiques 22. Les moyens de génération d'onde ultrasonore comportent quant à eux un ensemble d'éléments de transduction 23 (composé de 128 éléments) permettant d'imager le tissu pour étudier la propagation de l'onde élastique de cisaillement générée par les moyens d'excitation mécanique. L'ensemble d'éléments de transduction 23 est disposé entre les deux tringles 24.

Ce système permet la réalisation d'une cartographie 2D lors de l'estimation des propriétés viscoélastiques d'un tissu. Toutefois, sa conception présente de nombreux inconvénients. Notamment, le positionnement des tringles 24 de part et d'autre de l'ensemble d'éléments de transduction 23 :

- induit une largeur importante pour les moyens d'excitation mécanique qui rend le système 2 incompatible avec une exploration du foie entre les côtes d'un patient,
- peut, par ailleurs, poser des problèmes de désinfection du système 2.

Plus récemment il a été proposé, dans le document EP 3 315 074, une sonde pour élastographie transitoire comprenant :

- un boîtier,
- un (ou plusieurs) transducteur(s) ultrasonore ayant un axe de symétrie A,
- un (ou plusieurs) vibreur(s), chaque vibreur étant situé à l'intérieur du boîtier,
- un capteur de position couplé au boîtier,
- un circuit de rétroaction.

Le capteur de position est agencé pour mesurer le déplacement de la sonde. Le vibreur est apte à vibrer dans une gamme de fréquence comprise entre 1 Hz et 5kHz. Il est composé d'une partie fixe et d'une partie mobile dont la masse est supérieure ou égale à 25% de la masse totale de la sonde. La partie mobile est apte à se déplacer en translation le long d'une tige de guidage. Ce vibreur est agencé pour induire un mouvement du boîtier le long de l'axe de symétrie A du transducteur ultrasonore. Le circuit de rétroaction utilise le déplacement de la sonde pour commander le mouvement du (ou des) vibreur(s) à l'intérieur du boîtier et la forme d'une impulsion basse fréquence appliquée par la sonde.

Un inconvénient de la sonde décrite dans EP 3 315 074 concerne son encombrement important. Un autre inconvénient de la sonde selon EP 3 315 074 concerne la forte consommation en énergie électrique du vibreur, ce qui rend cette sonde inadaptée à une alimentation par batterie. Enfin, l'amortissement des vibrations générées par le (ou les) vibreur(s) peut varier en fonction de l'orientation de la sonde.

### 2. *Génération acoustique de l'onde de cisaillement*

Pour remédier aux inconvénients des appareil 1 et système 2 précités, il a été proposé de générer l'onde de cisaillement en utilisant des moyens acoustiques plutôt que des moyens mécaniques.

Différentes solutions basées sur la pression de radiation ultrasonore (qui consiste en une force volumique générée dans le milieu lors de la propagation d'une onde de compression par transfert de quantité de mouvement avec le milieu) ont ainsi été développées ces vingt dernières années.

Ces solutions sont basées sur le principe physique suivant. La focalisation d'un faisceau ultrasonore de forte intensité donne lieu à des effets non-linéaires résultant en une force s'exerçant sur le milieu au point de focalisation. Si l'énergie ultrasonore est délivrée sur un court instant (fraction de ms) elle résulte en une contrainte transiente ponctuelle qui génère une onde de cisaillement à symétrie sphérique partielle autour du point de focalisation.

Dans ces différentes solutions :

- les moyens d'excitation pour générer une onde de cisaillement, et
- les moyens de génération d'ondes ultrasonores haute fréquence pour imager le tissu

résultent de l'utilisation d'un seul composant, à savoir le réseau de transducteurs.

Ce réseau de transducteurs est alors apte à :

- dans un premier temps, générer une impulsion ultrasonore de forte intensité induisant la production d'une onde de cisaillement par effet non-linéaire,
- dans un deuxième temps, générer des d'ondes ultrasonores haute fréquence pour imager le tissu afin d'étudier la propagation de l'onde de cisaillement.

Toutefois, un inconvénient de cette technique concerne le fait que l'amplitude de l'onde de cisaillement générée est faible, et se propage sphériquement perdant rapidement l'amplitude nécessaire au déplacement des tissus en limitant la manière dont elle pénètre en profondeur dans le tissu, car c'est un effet du second ordre.

Pour remédier à cet inconvénient, il a été proposé d'émettre des ultrasons focalisés successivement à des profondeurs différentes pour créer des poussées par pression de radiation (ou *« pushs »* selon la terminologie anglo-saxonne). Les interférences constructives des ondes de cisaillement ainsi produites forment un « *cône de Mach »* supersonique (dans lequel la vitesse de la source est supérieure à celle de l'onde générée) et une onde conique de cisaillement est créée. Le réseau de transducteurs bascule ensuite dans un mode d'imagerie ultrarapide (où des ondes ultrasonores haute fréquence sont générées) pour suivre l'onde de cisaillement qui se propage dans le milieu.

Si cette technique permet l'obtention d'une onde conique de cisaillement en utilisant le transducteur d'imagerie ultrasonore lui-même, elle présente l'inconvénient d'être coûteuse en énergie.

### 3. *But de l'invention*

En résumé :

- les excitateurs mécaniques proposés dans la littérature présentent des caractéristiques d'ergonomie inenvisageables pour un produit commercial, et en particulier pour un produit destiné à l'évaluation de la fibrose hépatique,
- les excitateurs acoustiques (utilisant la force de radiation pour produire l'onde de cisaillement) présentent une efficacité électromécanique très faible non envisageable pour un produit ultraportable à faible consommation énergétique.

Un but de la présente invention est de proposer un système d'imagerie par ultrasons permettant de remédier à au moins l'un des inconvénients précités.

Plus précisément, un but de la présente invention est de proposer un système d'élastographie impulsionnelle 2D permettant, en minimisant l'énergie nécessaire à la production d'une onde de cisaillement, de mesurer les propriétés viscoélastiques d'un tissu.

## BREVE DESCRIPTION DE L'INVENTION

A cet effet, l'invention propose une sonde de mesure des propriétés viscoélastiques d'un milieu, par exemple un tissu biologique humain ou animal, tel qu'un foie, ladite mesure consistant à :

- générer au moins une onde élastique basse fréquence dans le milieu,
- simultanément à la génération de l'onde basse fréquence :

  - émettre des ondes ultrasonores haute fréquence, et
  - recevoir des échos acoustiques dus aux réflexions des ondes ultrasonores dans le milieu,

la sonde comportant :

- un boitier,
- un réseau de transducteurs, mécaniquement solidaire du boitier, pour l'émission des ondes ultrasonores haute fréquence et la réception des échos acoustiques,
- au moins un excitateur de vibration inertiel pour l'émission de l'au moins une onde élastique basse fréquence, ledit et au moins un excitateur incluant :

  - une partie fixe solidaire mécaniquement du réseau de transducteurs,
  - une partie mobile apte à se déplacer librement relativement à la partie fixe pour produire des vibrations afin de générer l'onde élastique basse fréquence,
  - au moins un ressort de rappel s'étendant entre la partie fixe et la partie mobile, ***remarquable en ce que*** la masse de la partie mobile est comprise entre 5 et 25% du poids total de la sonde, ***et en ce que*** le coefficient de raideur dudit et au moins un ressort de rappel est compris entre 300 kg.s$^2$ et 50 000 kg.s$^2$ de sorte que la fréquence de résonance de l'excitateur de vibration inertiel est sensiblement égale à la fréquence de l'onde élastique basse fréquence.

Ainsi, la masse (de la partie mobile de l'excitateur) et le coefficient de raideur (du (ou des) ressort(s) de rappel) sont choisis dans des plages correspondant à la famille des excitateurs pouvant être utilisés à leur fréquence de résonance respective pour émettre une onde élastique dans une gamme de fréquence d'intérêt.

Cette masse et ce coefficient de raideur sont en outre déterminés pour que la fréquence de résonance de l'excitateur soit égale à la fréquence de l'onde élastique. Plus précisément, si l'on souhaite que la sonde émette une onde élastique de fréquence « F », alors la masse « m » de la partie mobile de l'excitateur et le coefficient de raideur « k » du (ou des) ressort(s) sont choisis de sorte que $F = \frac{1}{2\pi}\sqrt{\frac{k}{m}}$. En pratique, pour dimensionner correctement la sonde, connaissant la fréquence « F » souhaitée pour l'onde élastique, le coefficient de raideur « k » (respectivement la masse « m ») est fixé dans la gamme comprise entre 300 kg.s$^2$ et 50 000 kg.s$^2$ (respectivement entre 5 et 25% du poids total de la sonde), et la masse « m » (respectivement le coefficient de raideur « k ») est calculée de sorte à satisfaire l'équation $m = \frac{k}{(2\pi F)^2}$ (respectivement $k = m(2\pi F)^2$).

On entend, dans le cadre de la présente invention par « *déplacement libre de la partie mobile de l'excitateur »,* le fait que la partie mobile se déplace sans subir de contrainte exercée par une force extérieure à l'excitateur, telle qu'une force exercée par l'utilisateur durant la préhension de la sonde. En particulier, les seules contraintes subies par la partie mobile lors de son déplacement sont la gravité et la (ou les) force(s) exercée(s) par l'excitateur, à savoir une force mécanique de rappel (exercée par un ressort de rappel) et une force électromagnétique dans le cas d'un excitateur à électroaimant.

Comme il ressortira de la description qui va suivre, la partie fixe solidaire mécaniquement du réseau de transducteurs peut être fixée :

- directement au réseau de transducteurs, c'est à dire être en contact physique avec le réseau de transducteurs), ou
- indirectement au réseau de transducteurs, c'est-à-dire être en contact physique avec un composant de la sonde autre que le réseau de transducteurs (comme par exemple un châssis ou le boîtier de la sonde), ce composant étant fixé (lui-même directement ou indirectement) au réseau de transducteurs.

Dans tous les cas, la solidarisation mécanique de la partie fixe au réseau de transducteurs induit l'absence de déplacement relatif de ces deux éléments l'un par rapport à l'autre.

Des aspects préférés mais non limitatifs de la sonde selon l'invention sont les suivants :

- chaque excitateur de vibration inertiel peut être dépourvu de glissière de guidage coopérant par friction avec une tige de guidage pour assurer le déplacement en translation de la partie mobile, ledit et au moins un ressort de rappel formant un guide pour le déplacement de la partie mobile relativement à la partie fixe ;
- la sonde peut également comprendre un contrôleur pour appliquer un signal d'excitation électrique permettant de piloter le déplacement de la partie mobile relativement à la partie fixe, la partie mobile comportant au moins un aimant permanent ;
- chaque excitateur de vibration inertiel peut également comprendre une masse inertielle supplémentaire répartie autour de l'aimant permanent ;
- la masse inertielle supplémentaire peut comprendre au moins une paroi latérale enroulée autour d'un axe d'enroulement s'étendant perpendiculairement à un segment de compression du ressort de rappel, ladite et au moins une paroi latérale entourant ledit segment de compression ;
- en variante, la masse inertielle supplémentaire peut présenter un axe de symétrie parallèle à un segment de compression du ressort de rappel et être répartie autour de l'aimant permanent ;
- en variante encore, la sonde peut également comprendre au moins une carte électronique d'acquisition, la masse inertielle supplémentaire consistant en ladite carte électronique solidaire mécaniquement de l'aimant permanent ;
- la sonde peut comprendre plusieurs excitateurs de vibration inertiel pilotés par le contrôleur, chaque excitateur comprenant un ressort de rappel respectif, chaque ressort de rappel ayant un coefficient de raideur différent des coefficients de raideur des autres ressorts de rappel ;
- l'excitateur de vibration inertiel peut également comporter une couche d'amortissement disposée entre la partie fixe et la partie mobile, ladite couche d'amortissement étant constituée dans un matériau absorbant les chocs ;
- le contrôleur peut être adapté pour appliquer un signal d'atténuation à chaque excitateur de vibration inertiel afin d'amortir une oscillation de la partie mobile de chaque excitateur de vibration inertiel relativement à la partie fixe dudit excitateur de vibration inertiel, ledit signal d'atténuation étant calculé en fonction d'une information représentative d'un déplacement relatif entre la sonde et le milieu d'intérêt (à partir du traitement des échos acoustiques reçus et/ou d'une mesure d'un courant électrique circulant dans l'excitateur),
- en variante, le contrôleur peut être programmé pour appliquer un signal d'excitation électrique à chaque excitateur afin d'induire l'oscillation de la partie mobile de chaque excitateur, sans application postérieure d'un signal d'atténuation, de sorte à laisser osciller librement la partie mobile de chaque excitateur.

## BREVE DESCRIPTION DES DESSINS

D'autres avantages et caractéristiques de la sonde selon l'invention ressortiront mieux de la description qui va suivre de plusieurs variantes d'exécution, données à titre d'exemples non limitatifs, à partir des dessins annexés sur lesquels :

- la figure 1 est une représentation schématique d'un appareil médical d'élastographie impulsionnelle ultrasonore de l'art antérieur,
- la figure 2 est une représentation schématique d'un système d'élastographie impulsionnelle de l'art antérieur,
- la figure 3 est une représentation schématique d'un premier mode de réalisation d'une sonde d'élastographie impulsionnelle ultrasonore selon l'invention,
- la figure 4 est une représentation schématique d'une première variante d'excitateur,
- la figure 5 est une représentation schématique d'une deuxième variante d'excitateur,
- la figure 6 est une représentation schématique d'un exemple d'excitateur selon la deuxième variante,
- la figure 7a est une représentation schématique d'une troisième variante d'excitateur,
- la figure 7b est une représentation schématique d'une quatrième variante d'excitateur,
- la figure 7c est une représentation schématique d'une cinquième variante d'excitateur,
- la figure 8 est un schéma de principe d'un excitateur,
- la figure 9 est une représentation schématique d'un deuxième mode de réalisation d'une sonde d'élastographie impulsionnelle ultrasonore selon l'invention,
- la figure 10 est une représentation schématique d'un troisième mode de réalisation d'une sonde d'élastographie impulsionnelle ultrasonore selon l'invention,
- la figure 11 est une représentation schématique d'un quatrième mode de réalisation d'une sonde d'élastographie impulsionnelle ultrasonore selon l'invention,
- la figure 12 est une représentation schématique d'une sixième variante d'excitateur,
- la figure 13 est une représentation en perspective d'un exemple de sonde selon l'invention,
- la figure 14 est une représentation en perspective d'un autre exemple de sonde selon l'invention.

## DESCRIPTION DETAILLEE DE L'INVENTION

On va maintenant décrire plus en détails différents modes de réalisation de la sonde selon l'invention en référence aux figures. Dans ces différentes figures, les éléments équivalents sont désignés par la même référence numérique.

### 1. *Généralités*

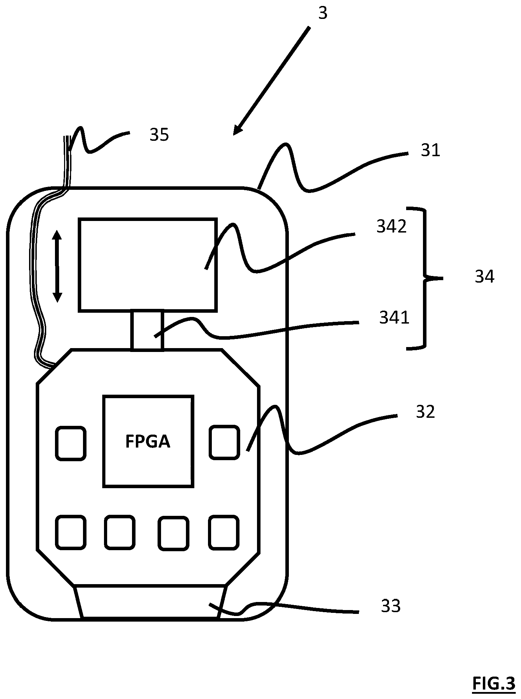

En référence à la figure 3, on a illustré un exemple de sonde d'élastographie impulsionnelle ultrasonore selon l'invention. Une telle sonde permet la mesure de l'élasticité d'un tissu ou d'un organe humain/animal.
La sonde 3 comprend :

- un boitier 31
- une carte électronique d'acquisition 32 (optionnelle),
- un réseau de transducteurs 33, et
- un excitateur de vibration inertiel 34 incluant une partie fixe 341 et une partie mobile 342 reliées entre elles par un ressort de rappel (non représenté sur la figure 3).

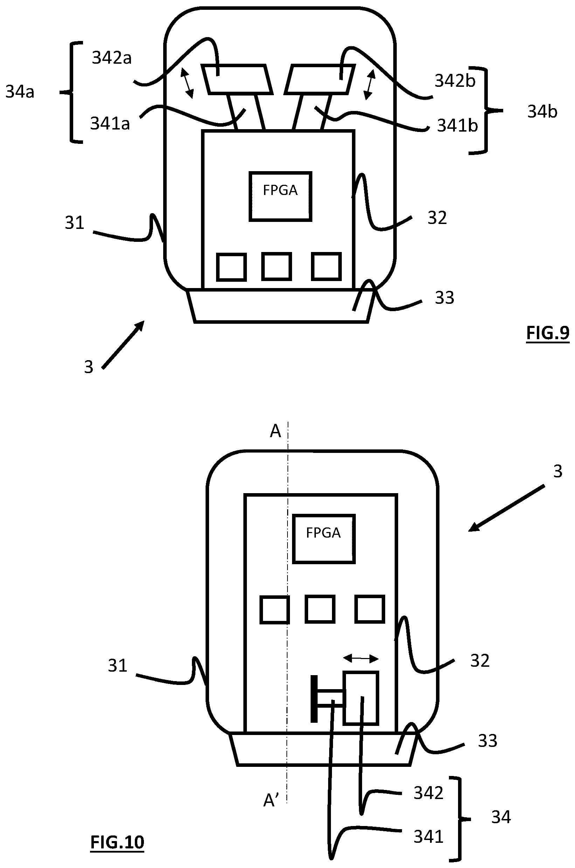

L'excitateur 34 permet de générer des vibrations. Comme il sera décrit plus en détails dans la suite, la partie fixe de l'excitateur 34 est mécaniquement solidaire du réseau de transducteurs 33 pour transmettre les vibrations à la sonde 3 afin de produire mécaniquement l'onde de cisaillement nécessaire à la mesure des propriétés viscoélastiques du tissu à analyser.
L'utilisation d'un excitateur de vibration inertiel 34 permet d'obtenir une sonde dans laquelle la masse de la partie mobile 342 générant les vibrations ne représente que 5 à 25% de la masse totale de la sonde, contrairement à la sonde selon EP 3 315 074 ainsi qu'aux appareil et système illustrés aux figures 1 et 2 dans lesquels l'essentiel de la masse de la sonde est concentré dans l'excitateur mécanique. On limite ainsi le poids et l'encombrement de la sonde selon l'invention.
Par ailleurs, le lecteur appréciera que, comme illustré aux figures 9, 10, 11 et 13, l'excitateur de vibration inertiel 34 n'est pas nécessairement agencé de manière à induire un mouvement de la sonde le long d'un axe de symétrie du réseau de transducteurs 33. En effet, dans de nombreux modes de réalisation, l'axe de symétrie du réseau de transducteurs 33 et l'axe de déplacement de la partie mobile de l'excitateur de vibration inertiel 34 ne sont pas confondus.

Ceci permet de limiter l'encombrement de la sonde selon l'invention.

L'utilisation d'un excitateur de vibration inertiel 34 permet en outre de générer une onde de cisaillement de puissance suffisante pour mesurer les propriétés viscoélastiques tissulaires tout en consommant une énergie inférieure à celle nécessaire aux solutions basées sur la pression de radiation ultrasonore.

### 1.1. Boitier

Le boitier 31 de la sonde 3 héberge les moyens d'acquisition de la sonde ainsi que les moyens de génération d'une onde de cisaillement. Plus précisément, l'éventuelle carte électronique 32, le réseau de transducteurs 33 et l'excitateur de vibration inertiel 34 sont logés dans le boitier 31.

Un avantage d'une telle sonde est que toutes les parties mobiles de la sonde (en particulier la partie mobile de l'excitateur de vibration inertiel 34) sont internes au boitier 31 et donc protégées. Ceci facilite la désinfection de la sonde 3. Vu de l'extérieur, la sonde est indéformable, ce qui résout bien des problèmes d'usabilité et facilite sa préhension par l'utilisateur.

La sonde comprend également des moyens de communication 35 (avec ou sans fil) pour envoyer les données acquises (éventuellement prétraitées et beamformées) à une unité de calcul (ordinateur distant et/ou tablette et/ou smartphone, etc.) pour la reconstruction d'images élémentaires d'un objet cible et/ou l'estimation et/ou l'affichage de l'élasticité de l'objet cible.

### 1.2. Réseau de transducteurs

Le réseau de transducteurs 33 comprend un jeu de « $n$ » transducteurs ultrasonores (« $n$ » étant un entier supérieur ou égal à un) disposés linéairement, ou en courbe, ou en cercles concentriques, ou en matrice.

Le réseau de transducteurs 33 permet d'émettre des ondes ultrasonores d'excitation vers un milieu à explorer (organe, tissu biologique, etc.), et de recevoir des échos acoustiques (i.e. ondes ultrasonores réfléchies par les différentes interfaces du milieu à explorer). Chaque transducteur consiste par exemple en une plaquette de matériau piézoélectrique de forme rectangulaire revêtue sur ses faces avant et arrière d'électrodes. De tels transducteurs sont connus de l'homme du métier et ne seront pas décrits plus en détails dans la suite.

Dans la variante de réalisation illustrée à la figure 3, tous les transducteurs du réseau sont utilisés à la fois en émission et en réception. Dans d'autres modes de réalisation, des transducteurs distincts peuvent être utilisés pour l'émission et la réception.

### 1.3. Carte électronique

La carte électronique optionnelle 32 est reliée au réseau de transducteurs 33. Elle permet de piloter les transducteurs du réseau, et de traiter les données acquises par les transducteurs du réseau. Plus précisément, la carte électronique 32 d'acquisition permet :

- de commander aux transducteurs l'émission d'ondes ultrasonores vers le milieu à explorer,
- de commander aux transducteurs la réception des échos réfléchis par les différentes interfaces du milieu à explorer,
- de prétraiter les signaux échos et de les transmettre à l'unité de calcul distante.

La carte électronique peut également comprendre un contrôleur pour piloter l'excitateur de vibration inertiel 34, comme il sera décrit plus en détails dans la suite.

### 1.4. Excitateur de vibration inertiel

L'excitateur de vibration inertiel 34 permet de faire vibrer la sonde 3 pour induire la génération d'une onde de cisaillement.

Plus précisément, la mise en mouvement de la partie mobile 342 - ou « masse inertielle » - de l'excitateur 34 dans la direction opposée à celle du déplacement désiré met le réseau de transducteurs 33 en mouvement dans l'autre sens, et produit l'onde de cisaillement souhaitée.

Avantageusement dans le cadre de la présente invention, c'est la partie fixe 341 de l'excitateur 34 qui est mécaniquement solidaire (« directement » ou « indirectement ») du réseau de transducteurs 33, contrairement aux solutions de l'art antérieur. En effet :

- dans l'appareil 1 d'élastographie illustré à la figure 1, le transducteur 11 est solidaire de la partie mobile de l'actionneur électrodynamique 12, l'autre partie, fixe, de l'actionneur étant tenu en main par l'utilisateur

- dans le système 2 d'élastographie illustré à la figure 2, l'ensemble d'éléments de transduction 23 est totalement dissocié des moyens d'excitation mécanique 22, 24 (la vibration générée par les moyens d'excitation mécanique 22, 24 n'est pas transmise à l'ensemble d'éléments de transduction 23).

Cet agencement particulier (utilisation d'un excitateur de vibration inertiel 34 dont la partie fixe 341 est reliée au réseau de transducteurs 33 pour le faire vibrer) permet de générer une onde de cisaillement de puissance acceptable avec :

- une sonde ayant un encombrement minimal (volume sensiblement identique à celui d'une sonde utilisant la force de radiation),
- une sonde consommant une énergie limitée pour générer l'onde de cisaillement. La source d'énergie nécessaire à l'excitation de l'actionneur 34 peut d'ailleurs être fournie par une batterie embarquée dans la sonde, lorsque le lien de communication 35 est sans fil.

En d'autres termes, la solution proposée présente les avantages des deux technologies de l'art antérieur sans leurs inconvénients respectifs, à savoir :

- un fort rendement de génération de l'onde de cisaillement (tout comme les solutions de génération mécaniques de l'onde de cisaillement), et
- un faible encombrement (tout comme les solutions de génération acoustiques de l'onde de cisaillement).
- un poids faiblement augmenté (pas plus de 25%, notamment 20%) par rapport à cette même sonde sans excitateur de vibration inertiel.

Dans le mode de réalisation illustré à la figure 3, tous les éléments disposés à l'intérieur du boîtier 31 sont mécaniquement solidaires à l'exception de la partie mobile 342.

Ces éléments mécaniquement solidaires (i.e. boitier 31, carte électronique 32, réseau de transducteurs 33 et partie fixe 341 de l'excitateur 34) constituent une seule masse, de l'ordre de 200 à 300 grammes.

La partie mobile 342 de l'excitateur 34 (formant une masse inertielle) a une masse comprise entre 25 et 50 grammes (et plus généralement de 5 à 25% du poids de la sonde). Le fait que la masse de la partie mobile 342 soit comprise entre 25 et 50 grammes (et plus généralement de 5 à 25% du poids total de la sonde) permet de disposer d'une masse inertielle :

- suffisamment importante pour garantir l'efficacité de génération de l'onde de cisaillement,
- suffisamment faible pour ne pas alourdir de façon excessive le poids de la sonde et ainsi assurer sa maniabilité.

L'excitateur de vibration inertiel est adapté pour être utilisé à sa fréquence de résonnance. Plus précisément :

- la masse de la partie mobile de l'excitateur et
- le coefficient de raideur du ressort de rappel (ou le coefficient de raideur total des ressorts de rappel)

sont choisis de sorte que la fréquence de résonance de l'excitateur corresponde à la fréquence souhaitée pour l'onde de cisaillement.

Notamment :

- la masse de la partie mobile est comprise entre 5 et 25% du poids total de la sonde, et

- le coefficient de raideur du (ou des) ressort(s) de rappel est compris entre 300 $kg.s^2$ et 50 000 $kg.s^2$, préférentiellement 1 000 $kg.s^2$ et 10 000 $kg.s^2$, et encore plus préférentiellement entre 4 000 $kg.s^2$ et 6 000 $kg.s^2$.

Le fait qu'il soit prévu de faire fonctionner l'excitateur à sa fréquence de résonance permet de limiter la quantité d'énergie nécessaire pour la génération des ondes de cisaillement. En limitant la consommation d'énergie de l'excitateur, il est alors possible d'alimenter la sonde par batterie.

*2. Excitateur*

*2.1. Première variante de réalisation de l'excitateur*

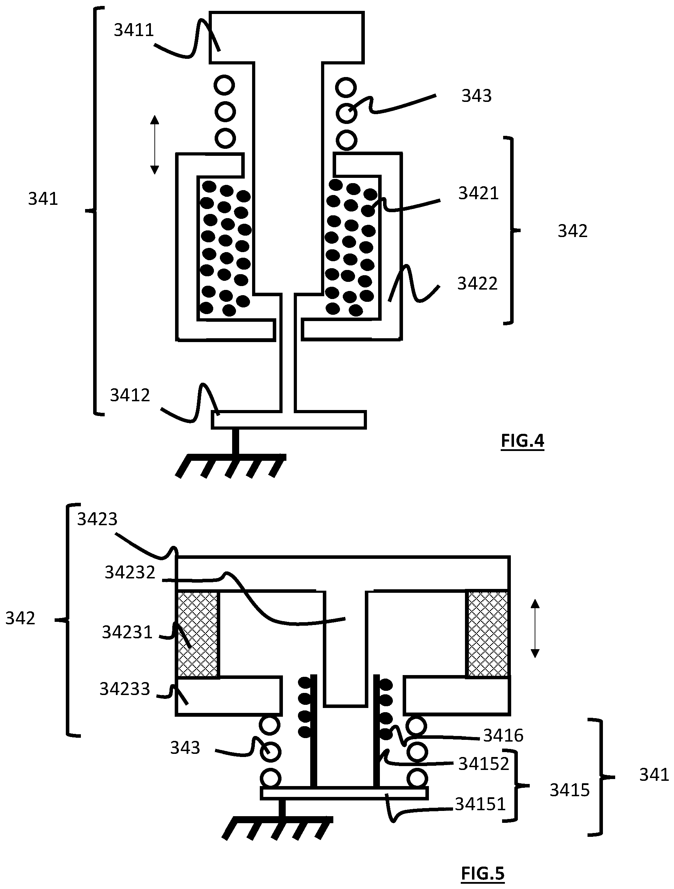

En référence à la figure 4, on a illustré une première variante de réalisation de l'excitateur (basée sur le principe de l'électroaimant et de la tige plongeuse) dans laquelle :

- la partie fixe 341 comporte une tige dite *« plongeuse »* incluant :

   ▪ une tête 3411 à l'une de ses extrémités, par exemple réalisée en Acier doux, et
   ▪ une embase 3412 à son autre extrémité, l'embase 3412 étant destinée à être fixée au boitier 31 directement ou indirectement (par exemple par l'intermédiaire de la carte électronique 32), et

- la partie mobile 342 est composée d'un électro-aimant incluant :

   ▪ un bobinage 3421 électriquement conducteur (par exemple en cuivre),
   ▪ un cœur magnétique 3422 (par exemple en acier doux) s'étendant autour de la tige et canalisant les lignes de champ magnétique,

- le ressort de rappel 343 s'étend entre la tête 3411 et le cœur magnétique 3422.

La tige plongeuse est montée coulissante à l'intérieur d'un conduit traversant ménagé dans le cœur magnétique 3422. Le principe de fonctionnement des excitateurs à électroaimant et tige plongeuse est le suivant.

Lorsqu'un courant électrique est appliqué dans le bobinage 3421, le matériau ferromagnétique de la tête 3411 est brusquement attiré vers le bas, ce qui induit un *« plongeon »* de la tige à l'intérieur du conduit traversant du cœur magnétique 3422. La tête 3411 se déplace vers le bas, et le ressort de rappel 343 se comprime. Dès que le bobinage 3421 n'est plus alimenté en courant électrique, le ressort de rappel 343 rappelle la tige coulissante en position *« haute »*.

Dans le cas d'espèce, l'embase 3412 de la tige plongeuse étant fixée (directement ou indirectement) au boitier 31 de la sonde 3, c'est le cœur magnétique 3422 qui se déplace vers la tête 3411 en comprimant le ressort 343 lors de l'application d'un courant électrique au bobinage 3421. Après interruption de ce courant électrique, le cœur magnétique 3422 revient en position *« basse »* du fait de la force appliquée par le ressort de rappel 343.

Il est alors possible d'obtenir un mouvement de va-et-vient du cœur magnétique 3422 selon que le courant électrique est appliqué ou interrompu dans le bobinage 3421. Ce mouvement de va-et-vient du cœur magnétique 3422 induit la vibration de l'ensemble de la sonde 3, ce qui permet de produire une onde de cisaillement.

De préférence, l'excitation électrique doit se rapprocher d'un Dirac (quelques millisecondes de durée).

Un inconvénient de ce type d'excitateur 34 est qu'il est difficile de contrôler finement le déplacement du cœur magnétique 3422 :

- sous l'effet de l'application du courant électrique, le cœur magnétique 3422 se déplace vers la tête 3411 de la tige en comprimant le ressort 343,
- la suite de la cinématique s'exerce en l'absence de force électromagnétique et se traduit par une oscillation du cœur magnétique 3422 avec une pulsation dépendant de sa masse et de la raideur du ressort 343.

C'est donc la raideur du ressort 343 qui va fixer la fréquence des mouvements du cœur magnétique 3422 : il n'y a pas de moyen de contrôler finement l'excitation, ni de l'amortir. Par ailleurs la force magnétique développée dans un tel système est très dépendante de l'enfoncement de la tige dans le cœur magnétique 3422, devenant maximale quand la tige arrive en contact avec le cœur magnétique 3422.

Un autre inconvénient de ce type d'excitateur concerne le guidage du déplacement en translation de la tige plongeuse. En particulier, les frottements liés au déplacement de la tige plongeuse à l'intérieur du conduit - formant glissière de guidage - induisent un amortissement par frottement. Un tel amortissement par frottement de la partie mobile présente l'inconvénient d'être incontrôlé, variable dans le temps et fortement dépendant de l'orientation de la sonde.

C'est pourquoi les inventeurs ont proposés d'autres variantes de réalisation (décrites ci-dessous) dans lesquelles l'excitateur de vibration inertiel est dépourvu de glissière de guidage coopérant en friction avec une tige pour assurer le déplacement en translation de la partie mobile. Ceci permet de disposer d'un excitateur de vibration inertiel qui a l'avantage de présenter un comportement oscillatoire intrinsèque invariable dans le temps et dans l'espace.

Dans ces différentes variantes décrites ci-dessous, le guidage du déplacement (en translation ou en rotation) de la partie mobile est assuré par un (ou des) ressort(s) de rappel 343 s'étendant entre la partie fixe 341 et la partie mobile 342.

### 2.2. *Deuxième variante de réalisation de l'excitateur*

En référence à la figure 5, on a illustré une deuxième variante d'excitateur autorisant plus de contrôle. Cet excitateur 34 fait encore appel aux forces électromagnétiques, mais travaille avec un système magnétique polarisé par un aimant permanent.

Plus précisément :

- la partie fixe 341 de l'excitateur 34 comprend :

    ○ une carcasse 3415 incluant :

        ▪ une embase 34151 destinée à être fixée (directement ou indirectement) sur le boitier 31,
        ▪ un support tubulaire 34152 s'étendant en saillie perpendiculairement à l'embase 34151, le support tubulaire 34152 incluant un conduit central,

    ○ une bobine d'activation électrique 3416 montée sur le support tubulaire 34152,

- la partie mobile 342 de l'excitateur 34 comprend une armature 3423 agencée pour entourer partiellement la carcasse 3415, l'armature 3423 incluant :

        ▪ un aimant permanent annulaire 34231, et
        ▪ un guide tubulaire 34232 axial en acier doux destiné à être monté coulissant à l'intérieur du conduit central du support tubulaire 34152,
        ▪ Une pièce polaire annulaire 34233 en acier doux entourant le support tubulaire 34152 et le bobinage 3416 pour définir un entrefer magnétique avec le guide tubulaire 34232,

- le ressort de rappel 343 s'étend entre l'embase 34151 et l'armature 34233.

Cet excitateur peut être piloté par un contrôleur (non représenté), par exemple intégré à la carte électronique 32. Ce contrôleur permet d'émettre un signal d'excitation électrique (de quelques millisecondes à quelques dizaines de millisecondes) pour alimenter la bobine d'activation 3416 de l'excitateur 34 dont le principe de fonctionnement est le suivant.

La bobine d'activation 3416 fixée mécaniquement au boitier 31 évolue dans l'entrefer de l'armature 3423 polarisée en continu par l'aimant permanent annulaire 34231.

Lorsque la bobine d'activation 3416 est alimentée en courant électrique (i.e. lorsque le contrôleur émet le signal électrique d'excitation), elle applique sur l'armature 3423 une force électromagnétique verticale proportionnelle au courant électrique, et dont le sens (vers l'embase 34151 ou vers l'opposée de l'embase 34151), dépend du sens du courant électrique.

Cette force induit le déplacement en translation de la partie mobile 342 le long du conduit central (du support tubulaire 34152) de la partie fixe 341. Plus précisément, en fonction du sens du courant électrique, cette force va :

- soit induire le déplacement en translation de la partie mobile vers l'embase 34151 de la partie fixe de sorte que le ressort de rappel 343 se comprime,
- soit induire le déplacement en translation de la partie mobile 342 dans une direction opposée à l'embase 34151 de la partie fixe 341 de sorte que le ressort de rappel 343 s'étire.

Après interruption du courant électrique, la partie mobile 342 revient dans sa position initiale, en oscillant du fait du ressort de rappel 343 qui se comprime et s'étire successivement jusqu'à revenir à sa position de repos. Il est alors possible d'obtenir un mouvement de va-et-vient de la partie mobile 342 selon que le courant électrique est appliqué ou interrompu dans la bobine d'activation 3416. Ce mouvement de va-et-vient de la partie mobile 342 induit la vibration de l'ensemble de la sonde 3 par réaction, ce qui permet de produire une onde de cisaillement lorsque la sonde 3 est au contact d'un tissu.

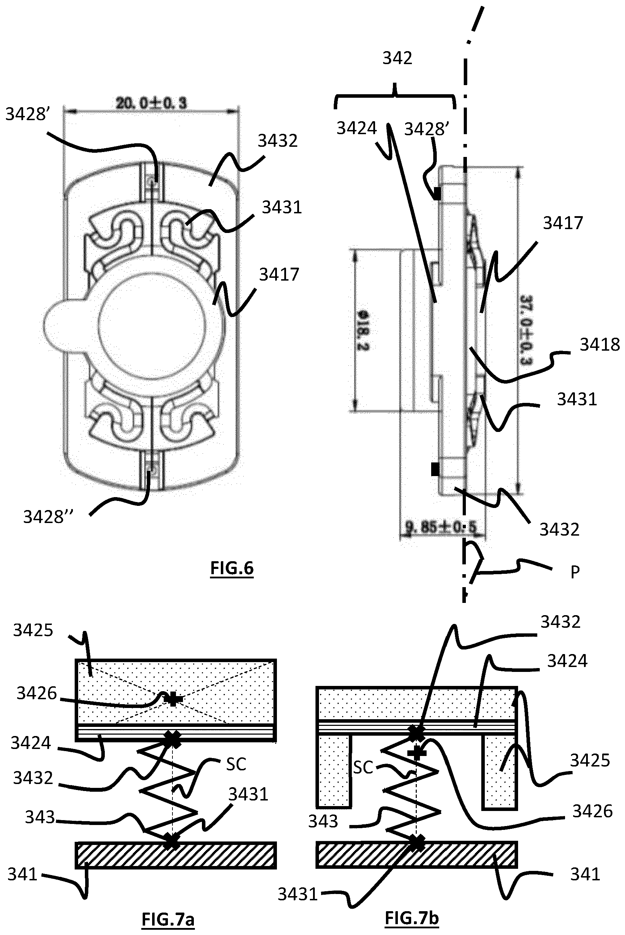

De tels dispositifs existent commercialement et s'appellent des excitateurs audios. A titre indicatif, on a illustré à la figure 6 un exemple d'excitateur audio du commerce (produit Tectonic référence TEAX 14C02-8) incluant les caractéristiques de l'excitateur inertiel de la figure 5.

En référence à la figure 6, cet excitateur audio comprend :

- un anneau circulaire 3417 formant embase, la face externe de l'anneau 3417 étant recouverte d'une couche adhésive,
- une bobine d'induction 3418 sur la face interne de l'anneau 3417,
- quatre pattes élastiques 3431 à retour de forme formant ressort de rappel et s'étendant en saillie vers l'extérieur de l'anneau 3417,
- un cadre 3432 sensiblement rectangulaire formant armature, l'extrémité de chaque patte élastique 3431 opposée à l'anneau 3417 étant reliée à un coin respectif du cadre 3432,
- un circuit magnétique polarisé par un aimant permanent 3424 fixé aux bords du cadre de plus grandes dimensions
- des connecteurs électriques 3428', 3428" pour raccorder électriquement la bobine d'induction 3418 à une source de courant (non représentée).

Un tel excitateur audio est adapté pour être fixé sur un support - tel qu'une plaque résonnante - et à le faire vibrer par inertie. Les dimensions de cet exemple d'excitateur audio du commerce sont compatibles avec la largeur et l'épaisseur des cartes électroniques utilisées dans les sondes existantes. La force générée sous un courant électrique de 1A est de 2.4 Newtons. La masse de la partie mobile d'un tel excitateur est de 12.8 grammes et oscille à 100 Hz (pour un poids total de la sonde de 235 grammes).

Ainsi pour faire osciller un tel excitateur à une fréquence de résonance de 50 Hz il est nécessaire d'augmenter la masse de la partie mobile à 51.2 grammes, par exemple en fixant une masse inertielle supplémentaire de 38.4 grammes à l'aimant permanent 3424.

Cette masse inertielle supplémentaire 3425 peut être fixée sur la face supérieure de l'aimant permanent 3424 tel qu'illustré à la figure 7a. Ceci induit une modification de la position du barycentre 3426 de la partie mobile 342 de l'excitateur 34, le barycentre 3426 étant alors situé en dehors d'un plan de fixation P de la partie mobile contenant le ou les points de fixation de la (ou des) extrémité(s) du (ou des) ressort(s) de rappel opposée(s) à la partie mobile. On entend, dans le cadre de la présente invention, par *« extrémités du ressort de rappel »* :

- si le ressort de rappel consiste en un unique élément élastique à retour de forme : les deux bouts de l'élément élastique,
- si le ressort de rappel consiste en une pluralité d'éléments élastiques à retour de forme, des points centraux situés à égale distance des bouts desdits éléments élastiques respectivement connectés à l'une ou l'autre des parties fixes et mobiles :

  ○ un premier point central situé à égale distance des bouts des éléments élastiques connectés à la partie fixe 341 formant une première extrémité du ressort de rappel 343, et
  ○ un deuxième point central situé à égale distance des bouts des éléments élastiques connectés à la partie mobile 342 formant une deuxième extrémité du ressort de rappel 343.

En variante et comme illustré à la figure 7b, la masse inertielle supplémentaire 3425 peut être répartie autour du ressort de rappel 343, de sorte que le barycentre 3426 de la partie mobile 342 soit localisé au voisinage du plan de fixation P de la partie mobile.

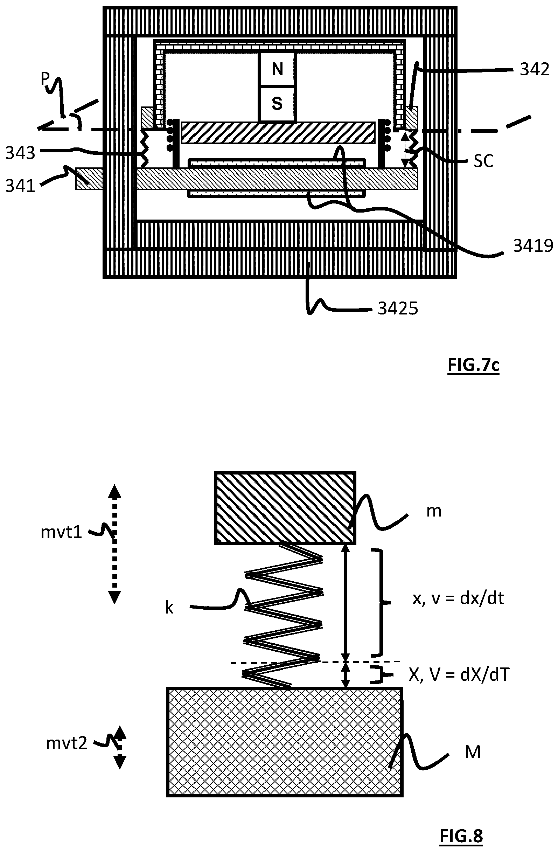

Dans le mode de réalisation illustré à la figure 7c, la masse inertielle supplémentaire est répartie tout autour de la partie mobile 342. Plus précisément dans ce mode de réalisation, la masse inertielle supplémentaire comprend une paroi latérale. Cette paroi latérale est enroulée autour d'un axe d'enroulement s'étendant perpendiculairement au segment de compression SC du (ou des) ressort(s) de rappel 343, la paroi latérale entourant le segment de compression SC. Ceci permet de limiter l'encombrement de l'excitateur. Par ailleurs dans ce mode de réalisation, l'excitateur comprend une couche d'amortissement 3419 disposée entre la partie fixe 341 et la partie mobile 342. Cette couche d'amortissement étant constituée dans un matériau absorbant les chocs. Ceci permet de limiter les risques de détérioration de l'excitateur en cas de chute de la sonde. En variante, la masse inertielle supplémentaire peut présenter un axe de symétrie parallèle à un segment de compression du ressort de rappel et être répartie autour de l'aimant permanent. Ceci permet de réduire la hauteur de l'excitateur (selon la direction de déplacement de la partie mobile), et donc de limiter l'encombrement de la sonde d'élastographie impulsionnelle ultrasonore.

Comme indiqué précédemment, à chaque application d'un signal d'excitation par le contrôleur, la partie mobile 342 de l'excitateur 34 oscille relativement à la partie fixe 341 jusqu'à revenir à sa position initiale de repos. Avantageusement, la vibration de la partie mobile 342 - notamment l'oscillation de la partie mobile provoquée par le ressort de rappel 343 jusqu'au retour à sa position initiale de repos - peut être atténuée (*« damping »*, selon la terminologie anglo-saxonne) par le contrôleur pour permettre un contrôle optimal de l'excitation mécanique. Cette atténuation peut être adaptative. En particulier, l'atténuation peut s'adapter aux variations de l'amortissement apporté par le contact entre la sonde et le corps du patient. Pour ce faire, il est nécessaire de connaitre les mouvements réels de la sonde. Cette connaissance des mouvements réels de la sonde peut être obtenue par différents moyens directs ou indirects. Par exemple la détermination de ces mouvements peut être réalisée sans utilisation de capteur de position spécifique :

- soit par le traitement des échos acoustiques reçus par le réseau de transducteurs (le film de déplacement ultrasonore pendant et après l'excitation mécanique peut donner une valeur précise du déplacement relatif entre la sonde et le tissu),
- soit en utilisant, après la phase d'excitation électrique, le signal amplifié par un amplificateur de courant de la bobine de l'excitateur. Ce signal est représentatif des mouvements relatifs de la partie mobile de l'excitateur 34 et de la sonde 31. Lorsqu'il est nul cela signifie que la partie mobile, et donc la sonde, n'oscille plus.

De préférence, le dispositif selon l'invention utilise une information représentative du déplacement relatif entre la sonde et le tissu (à partir du traitement des échos acoustiques reçus et/ou d'une mesure du courant électrique circulant dans la bobine de l'excitateur) pour calculer un signal permettant d'atténuer l'oscillation de la partie mobile. Ceci permet une atténuation plus efficace des oscillations de la partie mobile qu'avec une information représentative du déplacement absolu de la sonde.

Bien entendu, la vibration de la partie mobile 342 peut ne pas être atténuée. Dans ce cas, à chaque application d'un signal d'excitation par le contrôleur, la partie mobile 342 de l'excitateur 34 oscille librement. Cette libre oscillation induit la mise en mouvement de la sonde jusqu'à ce que la partie mobile soit revenue à sa position initiale de repos.

Le mouvement de la sonde (lié à l'oscillation libre de l'excitateur) peut être séparé de l'expression de l'avancée de l'onde de cisaillement par filtrage numérique des données acquises. En effet, dans le cadre de la présente invention, l'application d'un post-traitement permet de dissocier le mouvement de la sonde de l'expression du déplacement de l'onde de cisaillement Le fait de ne pas atténuer l'oscillation de la partie mobile permet de réduire l'énergie électrique consommée par la sonde. En effet, dans le cas d'une sonde dans laquelle les oscillations de la partie mobile de l'excitateur sont atténuées, il est nécessaire :

- d'utiliser un signal électrique d'excitation de plus forte puissance pour obtenir une onde de cisaillement dont l'énergie est équivalente à celle d'une onde de cisaillement obtenue à partir d'une sonde dans laquelle les oscillations de la partie mobile de l'excitateur ne sont pas atténuées,
- d'appliquer un signal électrique d'atténuation dont l'énergie est non nulle pour amortir les oscillations de la partie mobile.

*2.3. Remarques concernant les modes de réalisation des figures 7a à 7c*

Du fait de l'absence de glissière de guidage coopérant en friction avec une tige de guidage pour assurer le déplacement en translation de la partie mobile, les modes de réalisation de l'excitateur illustrés aux figure 7a à 7c ont l'avantage de présenter un comportement oscillatoire intrinsèque plus stable dans le temps et l'espace. Dans ces différents modes de réalisation, le (ou certains ou chaque) ressort(s) de rappel forme(nt) un guide de déplacement de la partie mobile, ce qui réduit les frottements et minimise le phénomène d'amortissement de l'oscillation.

De tels excitateurs sont excités à la fréquence de résonance, ce qui permet de limiter l'énergie consommée par l'excitateur pour générer l'onde de cisaillement, contrairement notamment au vibreur décrit dans EP 3 315 074.

*2.4. Théorie relative à l'invention*

Les différentes variantes de réalisation de l'excitateur 34 illustrées aux figures 4, 5, 6 et 7a, 7b peuvent être représentées par le schéma de principe illustré à la figure 8.

Dans cette figure :

- la masse « $m$ » représente la partie mobile 342 de l'excitateur 34 ; on note « $v$ » la vitesse instantanée de la masse « $m$ », et « $x$ » sa position,
- la masse « $M$ » représente :

  ◦ la partie fixe 341 de l'excitateur 34 ainsi que
  ◦ les autres composants de la sonde (boîtier, transducteurs, carte électronique, etc.) ;

  cette masse « $M$ » est typiquement comprise entre 200 et 300 grammes ; on note « $V$ » la vitesse instantanée de la masse « $M$ », et « $X$ » sa position,
- le coefficient de raideur « $k$ » représente le ressort de rappel 343.

Le lecteur appréciera qu'il est difficile de définir une masse effective associée à la masse « $M$ » réelle puisqu'une telle masse effective dépend notamment :

  ◦ de la force avec laquelle la sonde est serrée dans la main,
  ◦ du type de contact entre la main et la sonde (souplesse du matériau constituant les moyens de préhension de la sonde),
  ◦ de la force d'appui appliquée par la sonde sur le tissu.

Dans la suite des calculs, on considérera que la masse effective associée à la masse *« M »* est égale au double de la masse réelle de *« M »* (soit une masse effective comprise entre 400 et 600 grammes).

A un instant $t_0 = 0$, le ressort de rappel est à sa position de repos. Une force impulsionnelle est appliquée à la sonde qui tend à écarter les deux masses *« M »* et *« m »* pendant une durée de quelques millisecondes, et avec une énergie totale E.

Comme la même force agit sur les deux masses avec un signe opposé, il est possible d'écrire :

$$M*d(V)/d(t) = -m*d(v)/d(t)$$

Cette équation reste vraie après l'impulsion initiale lorsque le ressort de rappel réagit à l'écartement des deux masses puisqu'il exerce toujours des forces de même amplitude et de signe opposé sur les deux masses *« M »* et *« m »*. Il est donc possible d'écrire :

$$d(M*V + m*v)/dt = 0,$$

où : M*V + m*v = constante (qui n'est rien d'autre que la loi de conservation de la quantité de mouvement considérée dans le cadre de la théorie de la propulsion à réaction).

On obtient donc : v = M/m * V, et par intégration :

$$x = M/m*X \ (1)$$

La relation entre les termes de l'équation (1) indique que les vitesses et les déplacements sont dans le rapport inverse des masses. Cette relation permet en outre de vérifier une première cohérence du système : pour obtenir un déplacement de 0.1 mm de la masse *« M »*, il faut faire se déplacer de 1mm la masse inertielle *« m »* (qui est dix fois moins lourde que la masse *« M »*) ; un tel déplacement millimétrique est réaliste, en particulier en utilisant l'excitateur audio illustré à la figure 6.

Au niveau de l'énergie dépensée, il est possible d'écrire :

$$E = \tfrac{1}{2}*M*V^2 + \tfrac{1}{2}*m*v^2.$$

Notons $E_u$ l'énergie utile ; avec : $E_u = \tfrac{1}{2}*M*V^2$

Alors :

$$E = E_u*(1+m/M*(v/V)^2) = E_u(1+M/m)$$

L'efficacité énergétique $\xi$ vaut donc :

$$\xi = m \ / \ (m + M) \ (2)$$

Si les masses *« M »* et *« m »* sont dans un rapport 10, alors l'efficacité énergétique vaut 9%, ce qui est inférieur à celle du Fibroscan® (proche de 100%), mais bien supérieur à celle du *« push »* ultrasonore (probablement autour de 1/1000). Nous sommes donc encore là dans la cohérence du système.

On va maintenant étudier la valeur absolue de cette énergie.

Après l'impulsion initiale les deux masses *« M »* et *« m »* oscillent à une fréquence correspondant à une pulsation $\Omega$.

On peut démontrer facilement la relation classique entre la raideur du ressort, k, et $\Omega$ :

$$\Omega = \sqrt{(k*(1/m + 1/M))} \ (3)$$

Par ailleurs : $v_{Max} = \Omega * x_{Max}$

Où :

- $v_{Max}$ est la valeur maximale de la vitesse de la masse inertielle *« m »*, et
- $x_{Max}$ est la valeur maximale de l'éloignement de la masse inertielle *« m »*.

L'énergie fournie à la sonde satisfait donc l'équation suivante :

$$E= \tfrac{1}{2}*M*V^2 + \tfrac{1}{2}*m*v^2 = \tfrac{1}{2}*m/M*(m+M)* \Omega^2* x_{Max}^2$$

Dans le cas particulier où :

- la masse de *« m »* est le dixième de la masse de *« M »,*
- M=0.25 kg,
- la fréquence est 50Hz, et
- $x_{Max}$ vaut 1mm,

alors l'énergie fournie est : $E=0.5*0.1*0.275*(2*\pi*50*10^{-3})^2 = 1.4$ mJ

Cette énergie est très faible par rapport à la quantité d'énergie allouée au fonctionnement de la sonde (typiquement moins de cinq Watts). Même si l'on doit supposer que la masse effective est trois fois supérieure à celle de la sonde, et que par conséquent $x_{Max}$ doit aussi être trois fois supérieure à 1 mm, alors l'énergie nécessaire ne vaudra que dix fois plus, soit à peine plus que 10 mJ, ce qui reste encore très raisonnable.

En conclusion, les ordres de grandeur en termes :

- ∘ de masse inertielle,
- ∘ d'amplitude de déplacement de la masse inertielle, et
- ∘ d'énergie à apporter à la sonde

sont tous raisonnables et acceptables afin de générer l'onde de cisaillement.

L'utilisation d'un excitateur de vibration inertiel permet d'obtenir une sonde adaptée pour la génération d'une onde de cisaillement ayant :

- une efficacité énergétique bien supérieure à celle des solutions de type *« push »,* et
- un encombrement bien inférieur à celui des solutions utilisant des moyens d'excitation mécanique telles que le Fibroscan®.

### 3. *Exemples d'agencements de l'excitateur*

La figure 3 illustrait un premier mode de réalisation de la sonde. Comme le montre les figures 9 à 12, l'agencement de l'excitateur de vibration inertiel 34 dans la sonde 3 peut varier. On va maintenant décrire plus en détails différents modes de réalisation de la sonde en référence à ces figures 9 à 12.

### 3.1. *Deuxième mode de réalisation*

En référence à la figure 9, on a illustré un exemple de sonde 3 comprenant :

- un boitier 31
- une carte électronique d'acquisition 32,
- un réseau de transducteurs 33, et
- une paire d'excitateur de vibration inertiel 34a, 34b.

Chaque excitateur 34a, 34b comporte une partie fixe 341a, 341b et une partie mobile 342a, 342b reliées entre elles par un ressort de rappel. La partie fixe 341a, 341b de chaque excitateur 34a, 34b est fixée au bord de la carte électronique 32 opposé au bord connecté au réseau de transducteurs 33

Les excitateurs de vibration inertielle 34a, 34b peuvent être pilotés par un contrôleur, par exemple intégré à la carte électronique 32. Ce contrôleur permet d'appliquer un signal d'excitation électrique (de quelques millisecondes) pour induire la génération de vibrations par les excitateurs 34a, 34b.

L'avantage de ce système est que :

- si les deux excitateurs inertiels 34a, 34b sont pilotés en phase par le contrôleur (c'est-à-dire que le signal d'activation est appliqué simultanément aux deux excitateurs de vibration inertiels 34a, 34b), alors il est possible de générer un déplacement (vertical) en translation de la sonde,
- si par contre, les deux excitateurs inertiels 34a, 34b sont pilotés en opposition de phase par le contrôleur, alors il est possible de générer un déplacement en rotation de la sonde avec une composante principale horizontale dans le plan du transducteur.

Bien entendu, le lecteur aura compris que les excitateurs 34a, 34b peuvent être commandés dans d'autres configurations qu'en phase ou en opposition de phase, par exemple pour générer des déplacements à variation temporelle quelconque et prédéterminée. En outre, le lecteur appréciera que la sonde puisse comprendre plus de deux excitateurs de vibration inertiel disposés à des positions variables dans le boitier.

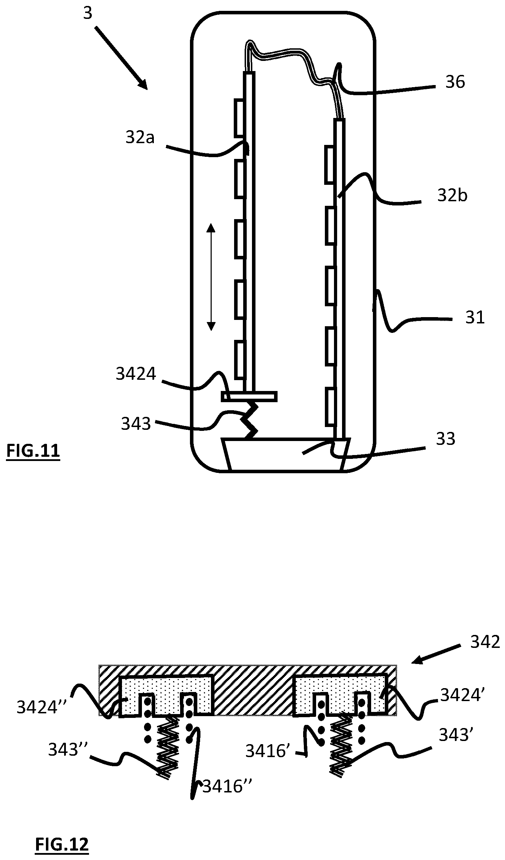

Le lecteur appréciera enfin que les excitateurs peuvent être agencés selon d'autres variantes de réalisation. Par exemple, dans le mode de réalisation illustré à la figure 12, la partie mobile 342 comprend :

- deux moteurs magnétiques incluant notamment chacun :

  ◦ une bobine d'activation 3416', 3416" et
  ◦ un aimant permanent 3424', 3424",

- deux ressorts de rappel 343', 343" de raideurs k', k" différentes (par exemple k' = 2xk"), chaque ressort étant associé à un moteur magnétique respectif.

On obtient ainsi deux oscillateurs couplés. Ceci permet d'explorer une gamme élargie de fréquences d'oscillation pour pouvoir évaluer la viscosité de l'objet cible.

### 3.2. *Troisième mode de réalisation*

En référence à la figure 10, on a illustré un autre exemple de sonde 3 comprenant :

- un boitier 31
- une carte électronique d'acquisition 32,
- un réseau de transducteurs 33, et
- un excitateur de vibration inertiel 34.

Dans ce mode de réalisation, l'excitateur de vibration inertiel 34 est :

- positionné à proximité du réseau de transducteurs 33 (i.e. au niveau du bord de la carte électronique raccordé au réseau de transducteur), et est
- orienté de sorte que la partie mobile 342 vibre en translation selon un axe transversal perpendiculaire à un axe longitudinal A-A' de la sonde 3,

contrairement au mode de réalisation illustré à la figure 3 dans lequel l'excitateur de vibration inertiel 34 est :

- positionné à distance du réseau de transducteurs 33 (i.e. au niveau du bord de la carte électronique opposé au réseau de transducteurs 33) et est
- orienté de sorte que la partie mobile 342 se déplace en translation parallèlement à l'axe longitudinal A-A' (c'est à dire perpendiculairement à un plan dans lequel s'étendent les transducteurs du réseau 33).

Dans ce troisième mode de réalisation, le positionnement et l'orientation de l'excitateur de vibration inertiel 34 permettent de générer des déplacements de la partie mobile 342 dans le plan des transducteurs du réseau 33.

### 3.3. *Quatrième mode de réalisation*

En référence à la figure 11, on a illustré un autre exemple de sonde dans lequel la masse inertielle supplémentaire 3425 fixée à l'aimant permanent 3424 de l'excitateur 34 consiste en une carte électronique 32a de la sonde 3.

Plus précisément dans ce mode de réalisation, la sonde comprend :

- un boitier 31,
- des première et deuxième cartes électroniques d'acquisition 32a, 32b, lesdites première et deuxième cartes électroniques étant électriquement raccordées l'une à l'autre grâce à des câbles de connexion souple 36,
- un réseau de transducteurs 33, et
- un excitateur de vibration inertiel 34 incluant :

○ une partie fixe (non représentée) solidaire mécaniquement du réseau de transducteurs 33,
○ une partie mobile incluant un circuit magnétique polarisé par un aimant permanent 3424, et
○ un ressort de rappel 343 entre les parties fixe et mobile.

Comme illustré à la figure 11, la première carte électronique 32a est solidaire mécaniquement de l'aimant permanent 3424 de l'excitateur 34. Ainsi, la première carte électronique 32a constitue la masse inertielle supplémentaire nécessaire pour induire la vibration de l'excitateur 34 à une fréquence de 50Hz. Ceci permet d'une part de limiter l'encombrement de la sonde 3 et d'autre part de limiter l'augmentation du poids de la sonde en utilisant l'un de ses composants pour former la masse inertielle supplémentaire.

La deuxième carte électronique 32b est quant à elle solidaire mécaniquement du réseau de transducteurs 33.

Le principe de fonctionnement est le suivant. Lorsqu'un signal d'activation (de quelques millisecondes) est appliqué à l'excitateur 34, l'aimant permanent 3424 et la première carte électronique 32a se déplacent en translation selon une direction opposée au réseau de transducteurs 33. Le ressort de rappel 343 s'étire. Après interruption du signal électrique d'activation, le ressort de rappel 343 applique une force sur l'aimant 3424 et la première carte électronique 32a pour les ramener vers le réseau de transducteurs 33. L'aimant permanent 3424 et la carte électronique 32a se déplacent en translation vers le réseau de transducteurs 33 et dépassent leur position de repos de sorte que le ressort de rappel 343 se comprime. Le ressort de rappel exerce alors sur l'aimant permanent 3424 (et la première carte électronique 32a) une force tendant à l'écarter du réseau de transducteur. Cette oscillation amortie se poursuit jusqu'à ce que l'aimant permanent et la carte électronique reviennent dans leur position de repos.

Pour poursuivre cette vibration, il est possible d'appliquer périodiquement le signal d'activation. On génère ainsi un train d'ondes de cisaillement dans le tissu lorsque la sonde est appliquée sur la peau du patient.

### 3.4. *Autres types d'excitateur*

Bien entendu, d'autres types d'excitateurs de vibration inertiel peuvent être utilisés pour permettre la génération de l'onde de cisaillement par la sonde. Par exemple la sonde incluant un excitateur de vibration inertielle 34 à moteur entrainant une masse excentrée.

### 4. *Conclusions*

Quel que soit l'excitateur 34 utilisé ou l'agencement retenu pour la sonde 3, le principe de fonctionnement de la sonde est le suivant.

L'excitateur de vibration actionneur 34 est activé pour induire la mise en mouvement de la sonde 3 en réaction à la mise en mouvement de sa partie mobile 342 et générer une onde élastique basse fréquence (l'onde de cisaillement) dans le tissu à analyser. Plus précisément, le contrôleur émet un signal d'excitation à l'excitateur 34.

Ce signal induit le déplacement de la (des) partie(s) mobile(s) relativement à la (aux) partie(s) fixe(s) (simultanément ou successivement, par exemple en opposition de phase). Lorsque ce signal (de quelques millisecondes) s'interrompt, la partie mobile revient à sa position d'origine en oscillant - ou non si le contrôleur émet un signal d'atténuation pour amortir l'oscillation de la partie mobile. Le mouvement vibratoire produit par l'excitateur est transmis à la sonde par l'intermédiaire de la partie fixe solidaire mécaniquement du réseau de transducteurs.

Durant la propagation de l'onde de cisaillement basse fréquence dans le tissu au contact, le réseau de transducteurs 33 émet et reçoit des ondes ultrasonores haute fréquence afin de permettre l'étude de la propagation de l'onde élastique basse fréquence.

Le mode de génération de l'onde de cisaillement proposé ci-dessus est efficace dans la transmission de l'énergie mécanique au tissu, puisque le réseau de transducteurs 33, au contact du tissu, est directement mis en mouvement par l'excitateur de vibration inertiel. Il permet en outre d'obtenir une sonde dont l'encombrement est minimisé.

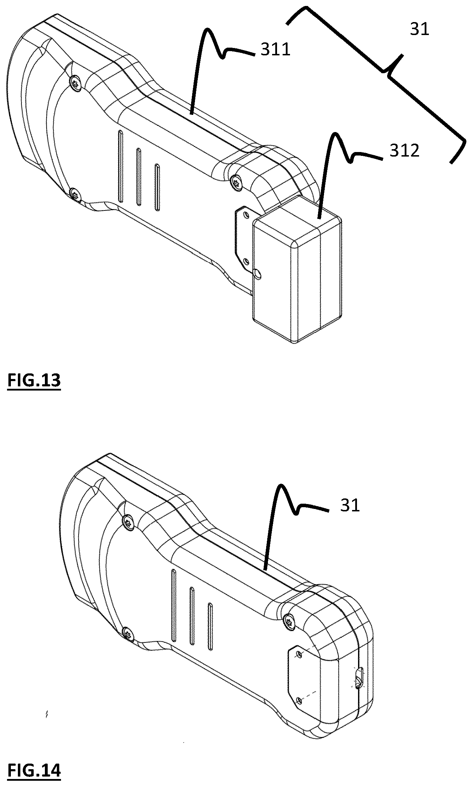

Différentes solutions peuvent être envisagées pour intégrer l'excitateur de vibration inertiel décrit précédemment dans la sonde. Par exemple, celui-ci peut être incorporé dans un réceptacle secondaire 312, comme illustré à la figure 13. Dans ce cas, le boitier 31 comprend :

- un réceptacle primaire 311 intégrant notamment l'éventuelle carte électronique d'acquisition et le réseau de transducteur,
- le réceptacle secondaire 312, le réceptacle secondaire 312 étant solidaire mécaniquement du réceptacle primaire 311.

En variante, l'excitateur de vibration inertiel peut être intégré dans le boitier 31 de la sonde 3 (figure 14). Dans tous les cas, l'excitateur de vibration inertiel est monté solidaire du réseau de transducteurs, et aucune partie mobile d'excitateur n'est au contact de l'utilisateur tenant la sonde, ce qui autorise sa libre oscillation. La prise en main de la sonde par l'utilisateur atténue l'amplitude de sa vibration telle qu'induite par l'excitateur, mais sans empêcher toutefois l'émission d'ondes de cisaillement au contact d'un patient du fait de l'élasticité des tissus de la main de l'utilisateur.

Le lecteur aura compris que de nombreuses modifications peuvent être apportées à l'invention décrite précédemment sans sortir matériellement des nouveaux enseignements et des avantages décrits ici.

Par conséquent, toutes les modifications de ce type sont destinées à être incorporées à l'intérieur de la portée des revendications jointes.

**Revendications**

1. Sonde de mesure des propriétés viscoélastiques d'un milieu, par exemple un tissu biologique humain ou animal, tel qu'un foie, ladite mesure consistant à :

   - générer au moins une onde élastique basse fréquence dans le milieu,
   - simultanément à la génération de l'onde basse fréquence :

      ◦ émettre des ondes ultrasonores haute fréquence, et
      ◦ recevoir des échos acoustiques dus aux réflexions des ondes ultrasonores dans le milieu,

   la sonde comportant :
   - un boitier (31),
   - un réseau de transducteurs (33), mécaniquement solidaire du boitier, pour l'émission des ondes ultrasonores haute fréquence et la réception des échos acoustiques,
   - au moins un excitateur de vibration inertiel (34) pour l'émission de ladite au moins une onde élastique basse fréquence, ledit au moins un excitateur incluant:

      ◦ une partie fixe (341) solidaire mécaniquement du réseau de transducteurs (33),
      ◦ une partie mobile (342) apte à se déplacer librement relativement à la partie fixe pour produire des vibrations afin de générer l'onde élastique basse fréquence,
      ◦ au moins un ressort de rappel (343) s'étendant entre la partie fixe (341) et la partie mobile (342),

   *caractérisé en ce que* la masse de la partie mobile (342) est comprise entre 5 et 25% du poids total de la sonde, *et en ce que* le coefficient de raideur dudit et au moins un ressort de rappel est compris entre 300 kg.s$^2$ et 50 000 kg.s$^2$ de sorte que la fréquence de résonance de l'excitateur de vibration inertiel (34) est sensiblement égale à la fréquence de l'onde élastique basse fréquence.

2. Sonde selon la revendication 1, *dans laquelle* chaque excitateur de vibration inertiel (34) est dépourvu de glissière de guidage coopérant par friction avec une tige de guidage pour assurer le déplacement en translation de la partie mobile, ledit et au moins un ressort de rappel formant un guide pour le déplacement de la partie mobile relativement à la partie fixe.

3. Sonde selon l'une quelconque des revendications 1 ou 2, *laquelle* comprend en outre un contrôleur pour appliquer un signal d'excitation électrique permettant de piloter le déplacement de la partie mobile (342) relativement à la partie fixe (341), la partie mobile comportant au moins un aimant permanent.

4. Sonde selon la revendication 3, *dans laquelle* chaque excitateur de vibration inertiel (34) comprend en outre une masse inertielle supplémentaire répartie autour de l'aimant permanent (3424).

5. Sonde selon la revendication 4, *dans laquelle* la masse inertielle supplémentaire comprend au moins une paroi latérale enroulée autour d'un axe d'enroulement s'étendant perpendiculairement à un segment de compression (SC) du ressort de rappel, ladite et au moins une paroi latérale entourant ledit segment de compression (SC).

6. Sonde selon la revendication 4, *dans laquelle* la masse inertielle supplémentaire s'étend autour d'un axe de symétrie parallèle à un segment de de compression (SC) du ressort de rappel, ladite masse étant répartie autour de l'aimant permanent (3424).

7. Sonde selon la revendication 4, *laquelle* comprend en outre au moins une carte électronique d'acquisition (32a), la masse inertielle supplémentaire consistant en ladite carte électronique (32a) solidaire mécaniquement de l'aimant permanent (3424).

8. Sonde selon la revendication 3, *laquelle* comprend plusieurs excitateurs de vibration inertiel (34a, 34b) pilotés par le contrôleur, chaque excitateur comprenant un ressort de rappel respectif (343', 343"), chaque ressort de rappel (343') ayant un coefficient de raideur différent des coefficients de raideur des autres ressorts de rappel (343").

9. Sonde selon l'une quelconque des revendications 1 à 8, *dans lequel* l'excitateur de vibration inertiel comporte en outre une couche d'amortissement (3419) disposée entre la partie fixe (341) et la partie mobile (342), ladite couche d'amortissement étant constituée dans un matériau absorbant les chocs.

10. Sonde selon l'une quelconque des revendications 3 à 9, *dans laquelle* le contrôleur est adapté pour appliquer un signal d'atténuation à chaque excitateur de vibration inertiel (34) afin d'amortir une oscillation de la partie mobile (342) de chaque excitateur de vibration inertiel (34) relativement à la partie fixe (341) dudit excitateur de vibration inertiel (34), ledit signal d'atténuation étant calculé en fonction d'une information représentative d'un déplacement relatif entre la sonde et le milieu d'intérêt a partir du traitement des échos acoustiques reçus et/ou d'une mesure d'un courant électrique circulant dans l'excitateur.

11. Sonde selon l'une quelconque des revendications 3 à 9, *dans laquelle* le contrôleur est programmé pour appliquer un signal d'excitation électrique à chaque excitateur afin d'induire l'oscillation de la partie mobile de chaque excitateur, sans application postérieure d'un signal d'atténuation, de sorte à laisser osciller librement la partie mobile de chaque excitateur.

**Patentansprüche**

1. Sonde zur Messung der viskoelastischen Eigenschaften eines Mediums, beispielsweise eines biologischen Gewebes eines Menschen oder eines Tieres, wie beispielsweise einer Leber, wobei die Messung darin besteht:

   - mindestens eine niederfrequente elastische Welle in dem Medium zu erzeugen,
   - gleichzeitig mit der Erzeugung der niederfrequenten Welle:

      ◦ hochfrequente Ultraschallwellen auszusenden, und
      ◦ akustische Echos zu empfangen, die durch die Reflexionen der Ultraschallwellen in dem Medium entstehen,

   wobei die Sonde aufweist:

      - ein Gehäuse (31),
      - ein mechanisch fest mit dem Gehäuse verbundenes Wandlernetzwerk (33) zum Aussenden der hochfrequenten Ultraschallwellen und zum Empfangen der akustischen Echos,
      - mindestens einen Trägheitsvibrationserreger (34) zum Aussenden der mindestens einen niederfrequenten elastischen Welle, wobei der mindestens eine Erreger umfasst:

         ◦ einen feststehenden Teil (341), der mechanisch fest mit dem Wandlernetzwerk (33) verbunden ist,
         ◦ einen beweglichen Teil (342), der imstande ist, sich relativ zum feststehenden Teil frei zu bewegen, um Vibrationen zur Erzeugung der niederfrequenten elastischen Welle zu erzeugen,
         ◦ mindestens eine Rückstellfeder (343), die sich zwischen dem feststehenden Teil (341) und dem beweglichen Teil (342) erstreckt,

      **dadurch gekennzeichnet, dass** die Masse des beweglichen Teils (342) zwischen 5 und 25% des Gesamtgewichts der Sonde liegt, und dass der Steifigkeitskoeffizient der und mindestens einen Rückstellfeder zwischen 300 kg.s$^2$ und 50.000 kg.s$^2$ liegt, so dass die Resonanzfrequenz des Trägheitsvibrationserregers (34) im Wesentlichen gleich der Frequenz der niederfrequenten elastischen Welle ist.

2. Sonde nach Anspruch 1, wobei jeder Trägheitsvibrationserreger (34) keine Führungsschiene aufweist, die reibschlüssig mit einer Führungsstange zusammenwirkt, um die Translationsbewegung des beweglichen Teils zu gewährleisten, wobei die und mindestens eine Rückstellfeder eine Führung für die Bewegung des beweglichen Teils relativ zum feststehenden Teil bildet.

3. Sonde nach einem der Ansprüche 1 oder 2, die ferner einen Controller zum Anlegen eines elektrischen Erregungssignals umfasst, das erlaubt, die Bewegung des beweglichen Teils (342) relativ zum feststehenden Teil (341) zu steuern, wobei der bewegliche Teil mindestens einen Permanentmagneten aufweist.

4. Sonde nach Anspruch 3, wobei jeder Trägheitsvibrationserreger (34) ferner eine um den Permanentmagneten (3424) verteilte zusätzliche Trägheitsmasse umfasst.

5. Sonde nach Anspruch 4, wobei die zusätzliche Trägheitsmasse mindestens eine Seitenwand umfasst, die um eine Wickelachse gewickelt ist, die sich senkrecht zu einem Kompressionssegment (SC) der Rückstellfeder erstreckt, wobei die und mindestens eine Seitenwand das Kompressionssegment (SC) umgibt.

6. Sonde nach Anspruch 4, wobei sich die zusätzliche Trägheitsmasse um eine Symmetrieachse parallel zu einem Kompressionssegment (SC) der Rückstellfeder erstreckt, wobei die Masse um den Permanentmagneten (3424) verteilt ist.

7. Sonde nach Anspruch 4, die ferner mindestens eine elektronische Erfassungskarte (32a) umfasst, wobei die zusätzliche Trägheitsmasse aus der elektronischen Karte (32a) besteht, die mechanisch fest mit dem Permanentmagneten (3424) verbunden ist.

8. Sonde nach Anspruch 3, die mehrere vom Controller gesteuerte Trägheitsvibrationserreger (34a, 34b) umfasst, wobei jeder Erreger eine jeweilige Rückstellfeder (343', 343") umfasst, wobei jede Rückstellfeder (343') einen Steifigkeitskoeffizienten aufweist, der sich von den Steifigkeitskoeffizienten der anderen Rückstellfedern (343'') unterscheidet.

9. Sonde nach einem der Ansprüche 1 bis 8, wobei der Trägheitsvibrationserreger ferner eine Dämpfungsschicht (3419) aufweist, die zwischen dem feststehenden Teil (341) und dem beweglichen Teil (342) angeordnet ist, wobei die Dämpfungsschicht aus einem stoßdämpfenden Material besteht.

10. Sonde nach einem der Ansprüche 3 bis 9, wobei der Controller geeignet ist, ein Dämpfungssignal an jeden Trägheitsvibrationserreger (34) anzulegen, um eine Schwingung des beweglichen Teils (342) jedes Trägheitsvibrationserregers (34) relativ zum feststehenden Teil (341) des Trägheitsvibrationserregers (34) zu dämpfen, wobei das Dämpfungssignal in Abhängigkeit von einer Information berechnet wird, die für eine relative Bewegung zwischen der Sonde und dem interessierenden Medium aus der Verarbeitung der empfangenen akustischen Echos und/oder einer Messung eines im Erreger fließenden elektrischen Stroms repräsentativ ist.

11. Sonde nach einem der Ansprüche 3 bis 9, wobei der Controller so programmiert ist, dass er an jeden Erreger ein elektrisches Erregungssignal anlegt, um den beweglichen Teil jedes Erregers in Schwingung zu versetzen, ohne anschließend ein Dämpfungssignal anzulegen, so dass der bewegliche Teil jedes Erregers frei schwingen kann.

**Claims**

1. A probe for measuring the viscoelastic properties of a medium, for example a human or animal biological tissue, such as a liver, said measurement consisting of:

   - generating at least one low-frequency elastic wave in the medium,
   - simultaneously with the generation of the low-frequency wave:

     ○ emitting high-frequency ultrasonic waves, and
     ○ receiving acoustic echoes due to the reflections of the ultrasonic waves in the medium,

   the probe including:

     - a housing (31),
     - a transducer array (33), mechanically integral with the housing, for emitting high-frequency ultrasonic waves and receiving acoustic echoes,
     - at least one inertial vibration exciter (34) for emitting said at least one low-frequency elastic wave, said at least one exciter including:

○ a fixed part (341) mechanically integral with the transducer array (33),
○ a mobile part (342) capable of moving freely relative to the fixed part to produce vibrations in order to generate the low-frequency elastic wave,
○ at least one return spring (343) extending between the fixed part (341) and the mobile part (342),

*characterised in that* the mass of the mobile part (342) is comprised between 5 and 25% of the total weight of the probe, *and in that* the stiffness coefficient of said and at least one return spring is comprised between 300 kg.s$^2$ and 50,000 kg.s$^2$ so that the resonance frequency of the inertial vibration exciter (34) is substantially equal to the frequency of the low-frequency elastic wave.

2. The probe according to claim 1, *wherein* each inertial vibration exciter (34) has no guiding slide cooperating by friction with a guide rod to ensure the translational movement of the mobile part, said and at least one return spring forming a guide for the movement of the mobile part relative to the fixed part.

3. The probe according to any one of claims 1 or 2, *which* further comprises a controller for applying an electrical excitation signal allowing to drive the movement of the mobile part (342) relative to the fixed part (341), the mobile part including at least one permanent magnet.

4. The probe according to claim 3, *wherein* each inertial vibration exciter (34) further comprises additional inertial mass distributed around the permanent magnet (3424).

5. The probe according to claim 4, *wherein* the additional inertial mass comprises at least one side wall wound around a winding axis extending perpendicularly to a compression segment (SC) of the return spring, said and at least one side wall surrounding said compression segment (SC).

6. The probe according to claim 4, *wherein* the additional inertial mass extends around an axis of symmetry parallel to a compression segment (SC) of the return spring, said mass being distributed around the permanent magnet (3424).

7. The probe according to claim 4, *which* further comprises at least one electronic acquisition card (32a), the additional inertial mass consisting of said electronic card (32a) mechanically integral with the permanent magnet (3424).

8. The probe according to claim 3, *which* comprises several inertial vibration exciters (34a, 34b) driven by the controller, each exciter comprising a respective return spring (343', 343"), each return spring (343') having a stiffness coefficient different from the stiffness coefficients of the other return springs (343").

9. The probe according to any one of claims 1 to 8, *wherein* the inertial vibration exciter further includes a damping layer (3419) disposed between the fixed part (341) and the mobile part (342), said damping layer being made of a shock-absorbing material.

10. The probe according to any one of claims 3 to 9, *wherein* the controller is adapted to apply an attenuation signal to each inertial vibration exciter (34) in order to dampen an oscillation of the mobile part (342) of each inertial vibration exciter (34) relative to the fixed part (341) of said inertial vibration exciter (34), said attenuation signal being calculated as a function of information representative of a relative movement between the probe and the medium of interest from the processing of the acoustic echoes received and/or from a measurement of an electric current flowing in the exciter.

11. The probe according to any one of claims 3 to 9, *wherein* the controller is programmed to apply an electrical excitation signal to each exciter in order to induce the oscillation of the mobile part of each exciter, without subsequent application of an attenuation signal, so as to allow the mobile part of each exciter to oscillate freely.

**FIG.1**

**FIG.2**

**FIG.3**

3411

341

343

3421

342

3422

3412

**FIG.4**

3423

34232

342

34231

34233

343

3416

34152

34151

3415

341

**FIG.5**

3428'

20.0±0.3

3432

3431

3417

3428''

**FIG.6**

342

3424  3428'

3417

Ø18.2

37.0±0.3

3418

3431

9.85±0.5

3432

P

3425

3426

3424

3432

SC

3431

343

341

**FIG.7a**

3432

3424

SC

343

3425

3426

341

3431

**FIG.7b**

**FIG.7c**

**FIG.8**

**FIG.9**

**FIG.10**

**FIG.11**

**FIG.12**

311

31

312

**FIG.13**

31

**FIG.14**

## RÉFÉRENCES CITÉES DANS LA DESCRIPTION

**Documents brevets cités dans la description**

- EP 3315074 A

**Littérature non-brevet citée dans la description**

- **SANDRIN**. *Shear Modulus Imaging with 2-D Transient Elastography*, 2002